# EUROPEAN PATENT APPLICATION

(11) **EP 4 702 943 A1**
(43) Date of publication of application: **04.03.2026**
(21) Application number: 25198440.7
(22) Date of filing: 27.08.2025
(51) Int. Cl.: A61B 34/00, A61B 18/14, A61B 34/10, A61B 34/20

(54) **ABLATION TAG CONNECTOR LINES AND ABLATION COUNT**

(30) Priority: 28.08.2024 US 202463687963 P; 21.08.2025 US 202519305910
(71) Applicant: Biosense Webster (Israel) Ltd., 2066717 Yokneam (IL)
(72) Inventor: TOV, Amatzia Eliyahu, 2066717 Yokneam (IL); ZAIDENBERG, Tomer, 2066717 Yokneam (IL); WATTAD, Maram, 2066717 Yokneam (IL); GOLDBERG, Stanislav, 2066717 Yokneam (IL); KLEMM, Ofer, 2066717 Yokneam (IL)
(74) Representative: Carpmaels & Ransford LLP

(57) **Abstract**

A method may comprise receiving, by a processor from one or more sensors, a three-dimensional (3D) position of each of a plurality of electrodes of a catheter during an ablation session, generating, by the processor, a 3D graphical representation identifying a position of each of the plurality of electrodes with a first indication, and connecting, by the processor, pairs of adjacent first indications with a second indication in the constellation-like 3D graphical representation. The second indications may indicate locations between electrodes where energy was delivered. The method may comprise displaying, by the processor on a display device, the 3D graphical representation comprising the first indications and the second indications. The method may comprise identifying pairs of adjacent first indications, that are not connected by a second indication, with a third indication, which may indicate locations between electrodes where energy was not delivered.

## Description

### CROSS-REFERENCE TO RELATED APPLICATIONS

This application claims the benefit of U.S. Provisional Application No. 63/687,963, filed August 28, 2025, the contents of which are incorporated herein by reference.

### TECHNICAL FIELD

The present application is related to generating display of information of ablation sessions. More particularly, systems and methods are disclosed for generating, visualizing, and displaying ablation electrode (tag) connector lines and ablation count information.

### BACKGROUND

In cardiac ablations using an electrophysiology (EP) mapping system, the use of a multi-electrode catheter, such as a bipolar Pulsed-Field Ablation (PFA) catheter, results in a large number of ablation tags being produced in close proximity in a three-dimensional (3D) space.

### SUMMARY

Implementations of the systems and methods described herein are directed to improving a display of information of ablations sessions. The method may comprise receiving, by a processor from one or more sensors, a three-dimensional (3D) position information of each of a plurality of electrodes of a catheter during an ablation session. The method may comprise generating, by the processor, a 3D graphical representation identifying a position of each of the plurality of electrodes with a first indication. The method may comprise connecting, by the processor, pairs of adjacent first indications with a second indication in the 3D graphical representation. The second indications may indicate locations between electrodes where ablation energy was delivered. The method may comprise displaying, by the processor on a display device, the 3D graphical representation comprising the first indications and the second indications. The method may comprise connecting pairs of adjacent first indications, that are not connected by a second indication, with a third indication. The third indications may indicate locations between electrodes where ablation energy was not delivered. The method may comprise displaying, by the processor on the display device, the 3D graphical representation comprising the first indications, the second indications, and the third indications. The first indications may be represented by at least at least a first distinguishing feature. The first distinguishing feature may be, for example a first color, a first shape, and/or a first shade or shading. The second indications may be represented by at least a second distinguishing feature. The second distinguishing feature may be, for example, a second color, a second shape, and/or a second shade or shading. The third indications may be represented by at least a third distinguishing feature. The third distinguishing feature may be, for example, a third color, a third shape, and/or a third shade or shading. A first distinguishing feature, a second distinguishing feature, and a third distinguishing feature may be different. The 3D graphical representation may comprise a gap between a pair of electrodes where no ablation energy was delivered. The pair of electrodes may not have a second indication connecting the pair of electrodes. The catheter may be a non-focal multi-electrode catheter. The catheter may be a bipolar Pulsed-Field Ablation (PFA) catheter. The first indications may be a sphere and the second indications may be a line. The method may comprise generating additional information. The method may comprise displaying the additional information along with the 3D graphical representation. The additional information may comprise at least one of: a count of an ablation session, an order of an ablation session, a timing of an ablation session, or a distance between a pair of electrodes. The method may comprise receiving, from a user, information that indicates which 3D graphical representation, from a plurality of generated 3D graphical representations, to display. The method may comprise selectively displaying the indicated 3D graphical representation.

According to one or more embodiments, the exemplary method embodiment may be implemented as an apparatus, a system, and/or a computer program product.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

A more detailed understanding may be had from the following description, given by way of example in conjunction with the accompanying drawings, wherein like reference numerals in the figures indicate like elements, and wherein:
FIG. 1 shows an example catheter-based electrophysiology mapping and ablation system according to one or more embodiments;
FIG. 2 is a block diagram of an example system for remotely monitoring and communicating patient biometrics according to one or more embodiments;
FIG. 3 is a system diagram of an example of a computing environment in communication with a network according to one or more embodiments;
FIG. 4 shows an example of a therapeutic catheter with a complex (non-linear) topology;
FIG. 5A shows an example 3D visualization of electrodes (e.g. tags) representing positions of ablation electrodes for multiple ablation sessions;
FIG. 5B shows a screenshot of a 3D visualization of electrodes (e.g. tags) representing positions of ablation electrodes for multiple ablation sessions;
FIG. 5C shows an example 3D visualization showing connections between electrodes (e.g. tags) that were part of ablation applications for each ablation session;
FIG. 5D shows a screenshot of a 3D visualization showing connections between electrodes (e.g. tags) that were part of ablation applications for each ablation session;
FIG. 5E shows an example 3D visualization identifying areas between electrodes where no ablation application was performed;
FIG. 5F shows a screenshot of a 3D visualization identifying areas between electrodes where no ablation application was performed;
FIG. 5G shows an example 3D visualization identifying an ablation session;
FIG. 5H shows a screenshot of a 3D visualization identifying an ablation session;
FIG. 6A shows an example 3D visualization identifying ablation sessions;
FIG. 6B shows a screenshot of a 3D visualization identifying ablation sessions;
FIG. 6C shows the example 3D visualization of FIG. 6A showing additional information;
FIG. 6D shows a screenshot of the example of FIG. 6B with additional information;
FIG. 6E shows an example of FIG. 6A identifying areas between electrodes where no ablation was performed;
FIG. 6F shows a screenshot of the example of FIG. 6B identifying areas between electrodes where no ablation application was performed;
FIG. 6G shows an example 3D visualization of ablation sessions that identify areas between electrodes where no ablation application was performed;
FIG. 6H shows a screenshot of a 3D visualization of ablation sessions that identify areas between electrodes where no ablation application was performed;
FIG. 7 shows an example method for generating and displaying information of ablation sessions; and
FIG. 8 shows an example method for generating and displaying information of ablation sessions.

### DETAILED DESCRIPTION

During a cardiac ablation procedure, for example a pulsed field ablation (PFA), electrodes of a multi-electrode catheter (e.g. a VARIPULSE^{™} catheter) may be activated to damage electrical pathways in tissue by forming non-conducting lesions. During a PFA ablation procedure, a chain of bipolar activations between electrodes (e.g. 1-2, 2-3, 1-3, and so no) form a single ablation session. Ablation electrodes may refer to the electrodes that were activated during a PFA ablation session to provide visual feedback to a healthcare provider (e.g. a physician). Some systems track the positions of the electrodes during each ablation session and provide a visualization of points or marks, sometimes referred to as tags or ablation tags (e.g. CARTO VISITAG^{™} Module Tag), in a three-dimensional (3D) environment (e.g. representing the tissue, blood vessel, or other environment in which the catheter is placed).

Due to the number of electrodes of a multi-electrode catheter and the number of ablation sessions, there may be visual clutter of the tags and it may be difficult for a physician to understand the relationship between the tags and correlate them to specific ablation sessions. It may be difficult for a physician to understand what areas were ablated and what areas need to be ablated. The lack of clear visualization and organization hinders the ability to analyze the multiple ablation sessions in a holistic manner.

Disclosed herein are systems and methods for providing clear visualization and organization by, for example, generating and displaying a 3D map visualization of ablation sites. The visualization connects tags created during each ablation session with lines, forming a 3D representation. Additional indications or information on or near the connecting lines, such as numbering, time stamping, or other indicia to provide information on the order and timing of each session may be included.

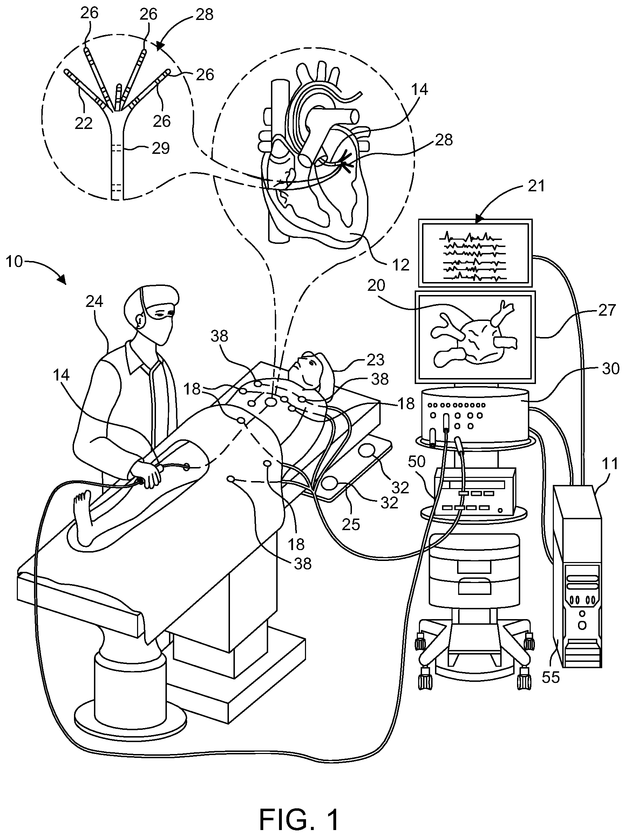

Reference is made to FIG. 1 showing an example system (e.g., medical device equipment and/or catheter-based electrophysiology mapping and ablation), shown as system 10, in which one or more features of the subject matter herein can be implemented according to one or more embodiments. The system 10, as illustrated, includes a recorder 11, a heart 12, a catheter 14, a model or anatomical map 20, an electrogram 21, a spline 22, a patient 23, a physician 24 (or a medical professional, healthcare provider, or clinician), a location pad 25, an electrode 26, a display device 27, a distal tip 28, a sensor 29, a coil 32, a patient interface unit (PIU) 30, an electrode skin patches 38, an ablation energy generator 50, and a workstation 55. Note further each element and/or item of the system 10 is representative of one or more of that element and/or that item. The example of the system 10 shown in FIG. 1 can be modified to implement the embodiments disclosed herein. The disclosed embodiments can similarly be applied using other system components and settings. Additionally, the system 10 can include additional components, such as elements for sensing electrical activity, wired or wireless connectors, processing and display devices, or the like

The system 10 includes multiple catheters 14, which are percutaneously inserted by the physician 24 through the patient's vascular system into a chamber or vascular structure of the heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters 14 may include catheters dedicated for sensing Intracardiac Electrogram (IEGM) signals, catheters dedicated for ablating and/or catheters dedicated for both sensing and ablating. The example catheter 14 that is configured for sensing IEGM is illustrated herein. The physician 24 brings the distal tip 28 of the catheter 14 into contact with the heart wall for sensing a target site in the heart 12. For ablation, the physician 24 would similarly bring a distal end of an ablation catheter to a target site for ablating.

The catheter 14 is an exemplary catheter that includes one and preferably multiple electrodes 26 optionally distributed over a plurality of splines 22 at the distal tip 28 and configured to sense the IEGM signals. The catheter 14 may additionally include the sensor 29 embedded in or near the distal tip 28 for tracking position and orientation of the distal tip 28. Optionally and preferably, position sensor 29 is a magnetic based position sensor including three magnetic coils for sensing three-dimensional (3D) position and orientation. The catheter 14 may be a Pulsed-Field Ablation (PFA) catheter. The catheter 14 may be a VARIPULSE^{™} catheter.

The sensor 29 (e.g., a position or a magnetic based position sensor) may be operated together with the location pad 25 including a plurality of magnetic coils 32 configured to generate magnetic fields in a predefined working volume. Real time position of the distal tip 28 of the catheter 14 may be tracked based on magnetic fields generated with the location pad 25 and sensed by the sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

The system 10 includes one or more electrode patches 38 positioned for skin contact on the patient 23 to establish location reference for the location pad 25 as well as impedance-based tracking of the electrodes 26. For impedance-based tracking, electrical current is directed toward the electrodes 26 and sensed at the patches 38 (e.g., electrode skin patches) so that the location of each electrode can be triangulated via the patches 38. Details of the impedance-based location tracking technology are described in US Patent Nos. 7,536,218; 7,756,576; 7,848,787; 7,869,865; and 8,456,182, which are incorporated herein by reference.

The recorder 11 displays the electrograms 21 captured with the electrodes 18 (e.g., body surface electrocardiogram (ECG) electrodes) and intracardiac electrograms (IEGM) captured with the electrodes 26 of the catheter 14. The recorder 11 may include pacing capability for pacing the heart rhythm and/or may be electrically connected to a standalone pacer.

The system 10 may include the ablation energy generator 50 that is adapted to conduct ablative energy to the one or more of electrodes 26 at the distal tip 28 of the catheter 14 configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The PIU 30 is an interface configured to establish electrical communication between catheters, electrophysiological equipment, power supply and the workstation 55 for controlling operation of the system 10. Electrophysiological equipment of the system 10 may include for example, multiple catheters 14, the location pad 25, the body surface ECG electrodes 18, the electrode patches 38, the ablation energy generator 50, and the recorder 11. Optionally and preferably, the PIU 30 additionally includes processing capability for implementing real-time computations of location of the catheters and for performing ECG calculations.

The workstation 55 includes memory, processor unit with memory or storage with appropriate operating software loaded therein, and user interface capability. The workstation 55 may provide multiple functions, optionally including (1) modeling the endocardial anatomy in three-dimensions (3D) and rendering the model or anatomical map 20 for display on the display device 27, (2) displaying on the display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

For instance, the system 10 can be part of a surgical system (e.g., CARTO^{®} system sold by Biosense Webster) that is configured to obtain biometric data (e.g., anatomical and electrical measurements of a patient's organ, such as the heart 12 and as described herein) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 12. Such locating may be done via an electrophysiological investigation during which electrical potentials are detected spatially resolved with a mapping catheter (e.g., the catheter 14) introduced into the chamber of the heart 12. This electrophysiological investigation, the so-called electro-anatomical mapping, thus provides 3D mapping data which can be displayed on the display device 27. In many cases, the mapping function and a treatment function (e.g., ablation) are provided by a single catheter or group of catheters such that the mapping catheter also operates as a treatment (e.g., ablation) catheter at the same time.

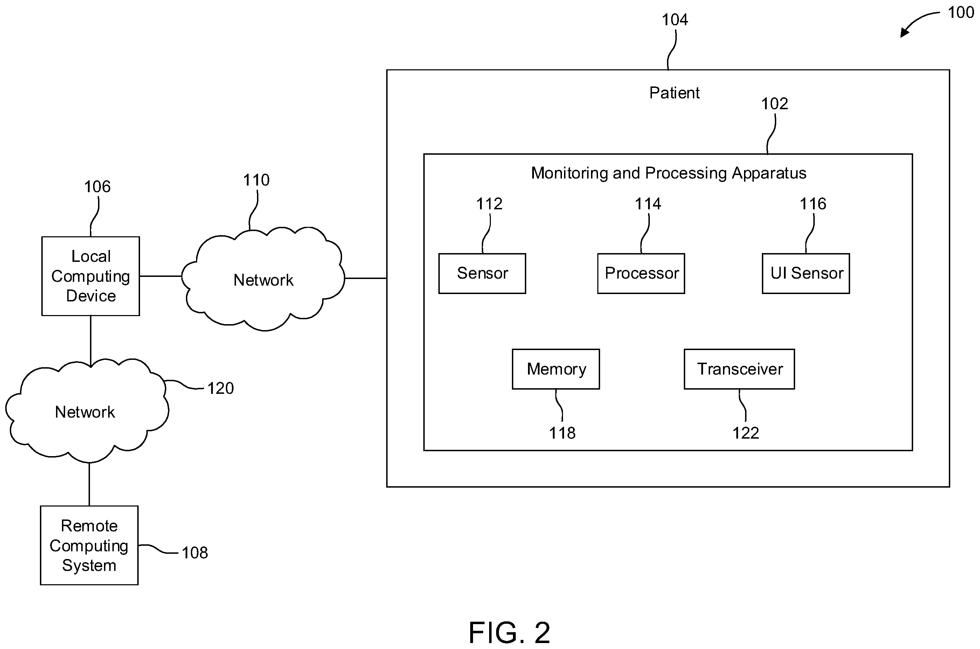

FIG. 2 is a block diagram of an example system 100 for remotely monitoring and communicating patient biometrics (i.e., patient data). In the example illustrated in FIG. 2, the system 100 includes a patient biometric monitoring and processing apparatus 102 associated with a patient 104, a local computing device 106, a remote computing system 108, a first network 110, a patient biometric sensor 112, a processor 114, a user input (UI) sensor 116, a memory 118, a second network 120, and a transmitter-receiver (i.e., transceiver) 122.

According to an embodiment, the patient biometric monitoring and processing apparatus 102 may be an apparatus that is internal to the patient's body (e.g., subcutaneously implantable), such as the catheter 14 of FIG. 1. The patient biometric monitoring and processing apparatus 102 may be inserted into a patient via any applicable manner including orally injecting, surgical insertion via a vein or artery, an endoscopic procedure, or a laparoscopic procedure.

According to an embodiment, the patient biometric monitoring and processing apparatus 102 may be an apparatus that is external to the patient, such as the electrode patches 38 of FIG. 1. For example, as described in more detail below, the patient biometric monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, the patient biometric monitoring and processing apparatus 102 may include both components that are internal to the patient and components that are external to the patient.

The single patient biometric monitoring and processing apparatus 102 is shown in FIG. 2. Example systems may, however, may include a plurality of patient biometric monitoring and processing apparatuses. A patient biometric monitoring and processing apparatus may be in communication with one or more other patient biometric monitoring and processing apparatuses. Additionally or alternatively, a patient biometric monitoring and processing apparatus may be in communication with the network 110.

One or more patient biometric monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other patient biometric monitoring and processing apparatuses 102. The additional information may be, for example, diagnosis information and/or additional information obtained from an additional device such as a wearable device. Each of the patient biometric monitoring and processing apparatus 102 may process data, including its own acquired patient biometrics as well as data received from one or more other patient biometric monitoring and processing apparatuses 102.

Biometric data (e.g., patient biometrics, patient data, or patient biometric data) can include one or more of local activation times (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, intracardiac electrocardiogram (IC ECG) data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treat any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

In FIG. 2, the network 110 is an example of a short-range network (e.g., local area network (LAN), or personal area network (PAN)). Information may be sent, via the network 110, between the patient biometric monitoring and processing apparatus 102 and the local computing device 106 using any one of various short-range wireless communication protocols, such as Bluetooth, Wi-Fi, Zigbee, Z-Wave, near field communications (NFC), ultraband, Zigbee, or infrared (IR).

The network 120 may be a wired network, a wireless network or include one or more wired and wireless networks. For example, the network 120 may be a long-range network (e.g., wide area network (WAN), the internet, or a cellular network,). Information may be sent, via the network 120 using any one of various long-range wireless communication protocols (e.g., TCP/IP, HTTP, 3G, 4G/LTE, or 5G/New Radio).

The patient biometric monitoring and processing apparatus 102 may include the patient biometric sensor 112, the processor 114, the UI sensor 116, the memory 118, and the transceiver 122. The patient biometric monitoring and processing apparatus 102 may continually or periodically monitor, store, process and communicate, via the network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 112 may include, for example, one or more sensors configured to sense a type of biometric patient biometrics. For example, the patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer or a microphone.

As described in more detail below, the patient biometric monitoring and processing apparatus 102 may be an ECG monitor for monitoring ECG signals of a heart (e.g., the heart 12). The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

The transceiver 122 may include a separate transmitter and receiver. Alternatively, the transceiver 122 may include a transmitter and receiver integrated into a single device.

The processor 114 may be configured to store patient data, such as patient biometric data in the memory 118 acquired by the patient biometric sensor 112, and communicate the patient data, across the network 110, via a transmitter of the transceiver 122. Data from one or more other patient biometric monitoring and processing apparatus 102 may also be received by a receiver of the transceiver 122, as described in more detail below.

According to an embodiment, the patient biometric monitoring and processing apparatus 102 includes a UI sensor 116 which may be, for example, a piezoelectric sensor or a capacitive sensor configured to receive a user input, such as tapping or touching. For example, the UI sensor 116 may be controlled to implement a capacitive coupling in response to tapping or touching a surface of the patient biometric monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from the patient biometric monitoring and processing apparatus 102 when a gesture is detected.

The local computing device 106 of the system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via the network 120. Alternatively, the local computing device 106 may be a stationary or standalone device, such as a stationary base station including, for example, modem and/or router capability, a desktop or laptop computer using an executable program to communicate information between the patient biometric monitoring and processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, the remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

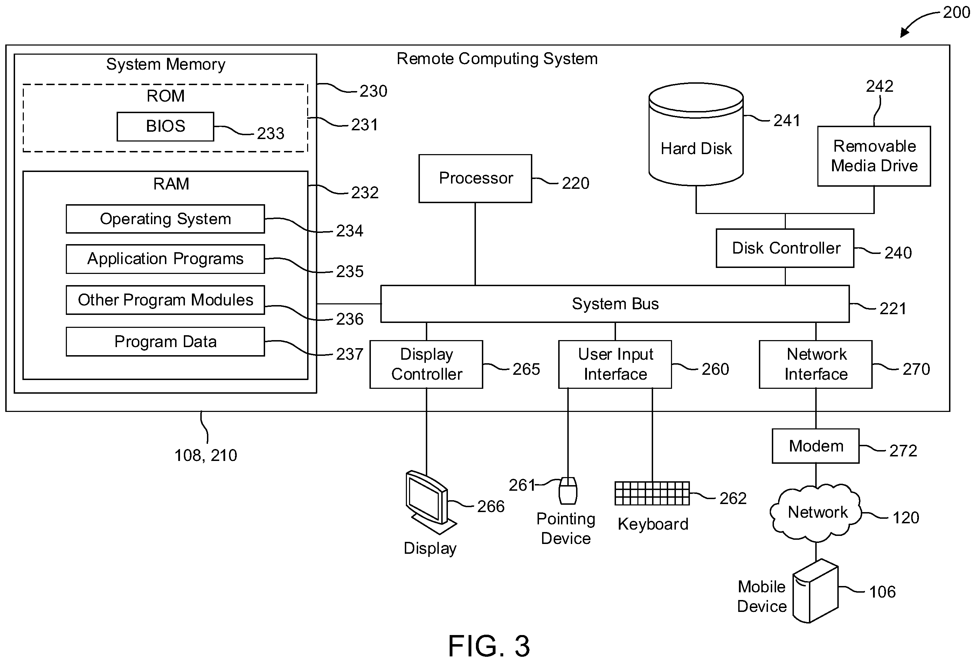

FIG. 3 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 3, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm-driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 3, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or all of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multi-processing arrangement to execute one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGS. 2 and 3, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 210 and other computers (e.g., local computing device 106).

A catheter ablation based treatment may include mapping the electrical properties of heart tissue, especially the endocardium and the heart volume, and selectively ablating cardiac tissue by application of energy. Cardiac mapping, for example creating a map of electrical potentials (a voltage map) of the wave propagation along the heart tissue or a map of arrival times (a local time activation (LAT) map) to various tissue located points, may be used for detecting local heart tissue dysfunction. Ablations, such as those based on cardiac mapping, can cease or modify the propagation of unwanted electrical signals from one portion of the heart to another.

The ablation process damages the unwanted electrical pathways by formation of non-conducting lesions. Cardiac ablation may rely on the use of three dimensional (3D) mapping systems, for example CARTO^{®}3 3D mapping system, produced by Biosense Webster, Inc. (Diamond Bar, Calif). The 3D maps can provide multiple pieces of information including visualization of tags (e.g. on a display or monitor) representing the location of electrodes of a multi-electrode catheter for an ablation session.

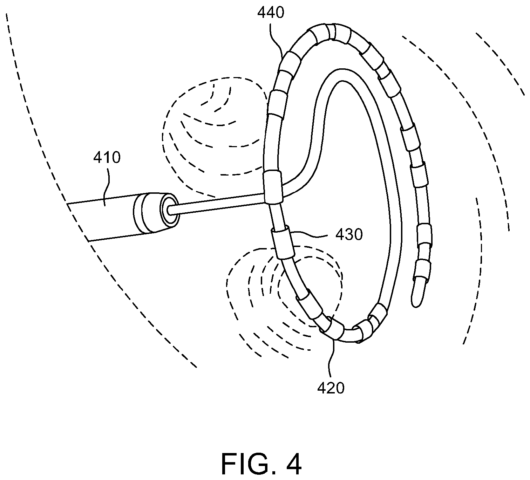

FIG. 4 shows an example of a therapeutic catheter 410 with a complex (non-linear) topology. The catheter 410 may be a non-focal catheter. The catheter 410 may have, for example, a lasso or loop shape with multiple electrodes. The catheter may have bipolar electrodes. A VARIPULSE^{™} catheter is an example of a multiple-electrode non-focal PFA catheter with a lasso or loop shape and may comprise a plurality, for example, ten, ablation electrodes. The catheter 410 includes multiple electrodes, for example 420, 430, and 440, that may be used to map a cardiac area. The catheter 410 may be fully or partially elastic such that it can twist, bend, and or otherwise change its shape based on a received signal and/or based on application of an external force (e.g., cardiac tissue) on the catheter 410. The distance between electrodes may be known.

Some systems may illustrate or display the results of ablation sessions via a point cloud of marks or tags (e.g. CARTO VISITAG^{™} Module Tag) in a 3D position representing positions of each electrode during ablation across the plurality of ablation sessions. An ablation or ablation session as discussed herein may be performed using, for example, a variable lasso or looped shaped PFA ablation catheter, for example a VARIPULSE^{™} PFA catheter, with multiple electrodes.

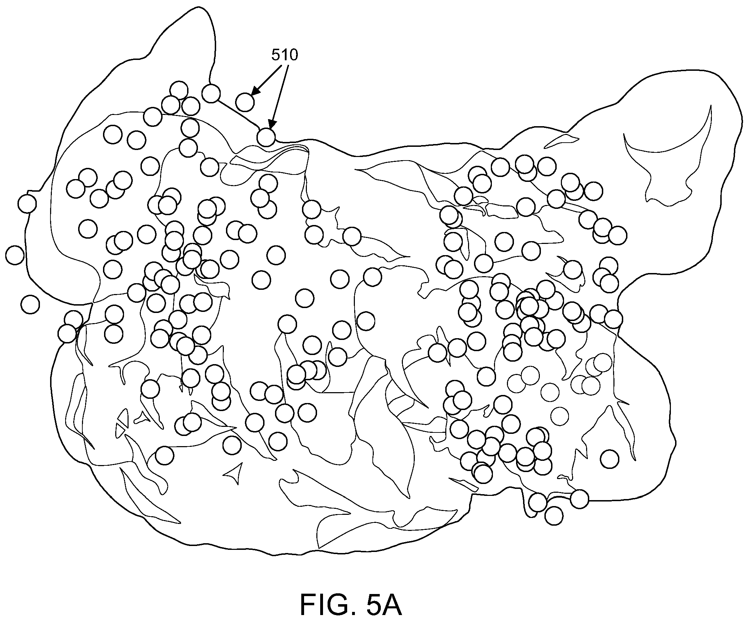

FIG. 5A shows an example 3D visualization of electrodes (e.g. tags or ablation tags) representing positions of ablation electrodes for multiple ablation sessions. FIG. 5A shows a cluttered 3D point cloud of tags 510 generated from a plurality of ablation sessions. Each tag 510, which may be variously referred to as a point, position, electrode position, mark, tag, ablation tag, (e.g., CARTO VISITAG^{™} Module Tag), or by any other similar term, is represented by a sphere or circle in FIG. 5A. However, the spheres or circles shown in FIG. 5A are just one example and the tags 510 may be represented by any distinguishing feature such as a geometric shape, a shade or shading, and/or color. The tags 510 indicate positions of ablation electrode that were part of an ablation application for each ablation session (e.g. bipolar electrodes of a non-focal catheter). In implementations using point clouds as shown in the example, it may be difficult for a physician to understand the effect of an ablation session or sessions, or to be able to distinguish between one ablation and another. For example, it may be difficult for a physician to understand the relationship between the tags (e.g. CARTO VISITAG^{™} Module Tag) and correlate them to a specific ablation session or sessions. It may also be difficult for a physician to understand what areas were ablated and what areas need to be ablated. There is no indication as to which tags were created during the same ablation session. The lack of clear visualization and organization hinders the ability to analyze the multiple ablation sessions in a holistic manner.

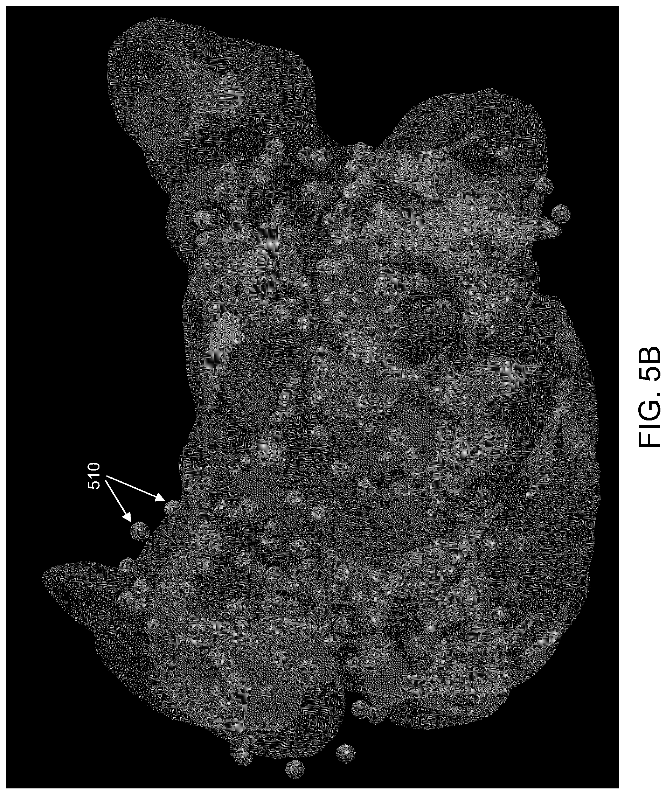

FIG. 5B is a screenshot of a 3D visualization of electrodes (e.g. tags or ablation tags) representing positions of ablation electrodes for multiple ablation sessions. FIG. 5B shows a cluttered 3D point cloud of tags generated from a plurality of ablation sessions. Each tag 510, which may be variously referred to as a point, position, electrode position, mark, tag, ablation tag, (e.g., CARTO VISITAG^{™} Module Tag), or by any other similar term, is represented by a purple sphere, circle, or dot in the screenshot of FIG. 5B. However, the purple spheres shown in FIG. 5B are just one example and the tags 510 may be represented by any distinguishing feature such as a geometric shape, a shade or shading, and/or color. The tags indicate positions of ablation electrode that were part of an ablation application for each ablation session (e.g. bipolar electrodes of a non-focal catheter). In implementations using point clouds as shown in the example, it may be difficult for a physician to understand the effect of an ablation session or sessions, or to be able to distinguish between one ablation and another. For example, it may be difficult for a physician to understand the relationship between the tags (e.g. CARTO VISITAG^{™} Module Tag) and correlate them to a specific ablation session or sessions. It may also be difficult for a physician to understand what areas were ablated and what areas need to be ablated. There is no indication as to which tags were created during the same ablation session. The lack of clear visualization and organization hinders the ability to analyze the multiple ablation sessions in a holistic manner.

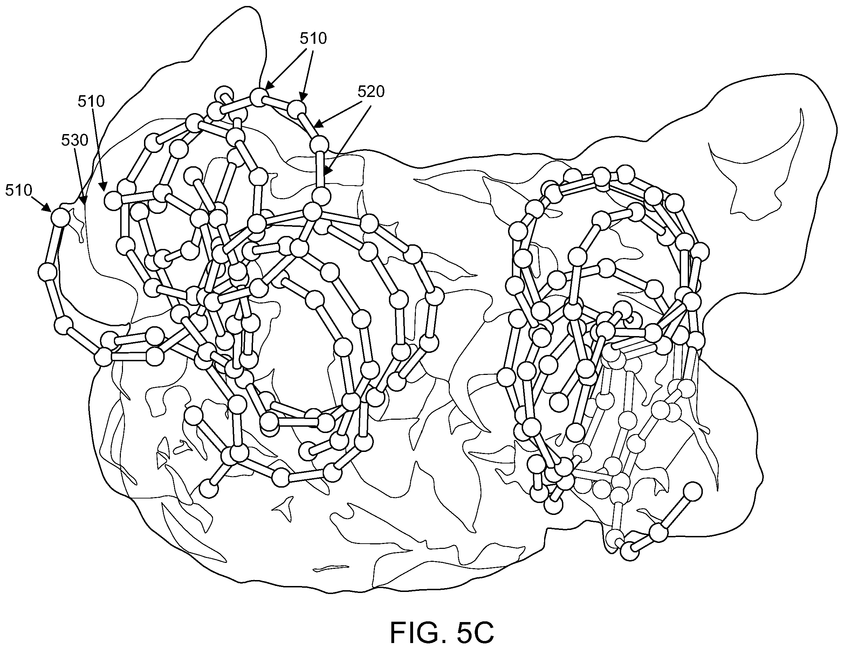

FIG. 5C is an example 3D visualization showing connections between electrodes (e.g. tags or ablation tags) that were part of ablation applications for an ablation session. In FIG. 5C a 3D point cloud of tags is shown which may be generated from a plurality of ablation sessions according to one or more embodiments. For an ablation session, the tags 510 (i.e. spheres or circles), which represent positions of ablation electrodes that were part of an ablation application for each ablation session, are connected by, for example, lines or rectangles 520. The lines 520 may be any shape, color or shade of color, or any shading. A line is one example, but any distinguishing feature such as a shape, shade, or color may be used to indicate a connection between electrodes (i.e., indicating area of ablation). The feature representing the lines (e.g., shape, color, shade of color, or shading) 520 may be different than the feature (e.g., shape, color, shade of color, or shading) representing the tags 510. The features representing the tags 510 (e.g., shape, color, shade of color, or shading) and/or the features representing the line 520 (e.g., shape, color, shade of color, or shading) of one ablation session may be different than the features of a different ablation session. The connecting of multiple tags together via lines 520 forms what may be referred to as a bracelet shape in this example of using a PFA non-focal lasso shaped catheter. However, if the ablation was performed by a different catheter, the connecting of the tags may form a different shape and may be referred differently. By connecting the tags, which represent positions of bipolar electrodes, showing that a PFA bipolar ablation application was performed between them, it helps to organize information, clear clutter on the screen, and visually separate ablation session from each other. This helps a physician identify which tags belong to a same ablation session. Using a catheter with bipolar electrodes (e.g. VARIPULSE^{™} catheter), ablation is performed at and between the electrodes, so the resulting bracelet visualizes areas where ablation energy was delivered in the proximity of ablation targets (e.g. energy was delivered or transferred between electrodes). The gap 530 indicates an area where no energy was applied or transferred between that pair of electrodes.

As shown in FIG. 5C, not all the tags 510 of an ablation session are connected by a line 520 (i.e. there is a gap 530 between two electrodes of a bracelet (e.g., between distal and proximal electrodes in a ring-link catheter such as VARIPULSE^{™})). This may occur in a case of a bipolar PFA application in a PFA ablation session where a pair of electrodes were not part of the bipolar PFA application (e.g. there was no energy applied or transferred between that pair of electrodes).

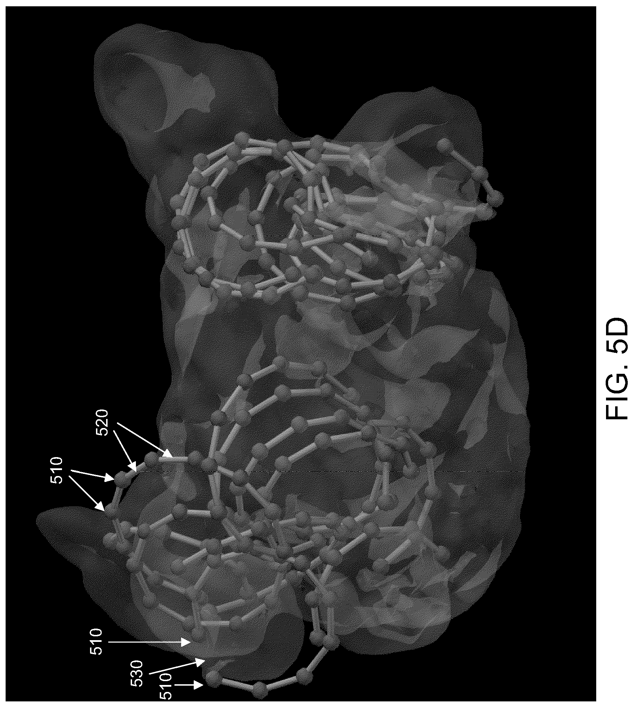

FIG. 5D is a screenshot showing connections between electrodes (e.g. tags or ablation tags) that were part of ablation applications for each ablation session. In FIG. 5D, a 3D point cloud of tags is shown which may be generated from a plurality of ablation sessions according to one or more embodiments. For an ablation session, the tags 510 (i.e. purple spheres or circles), which represent positions of ablation electrodes that were part of an ablation application for each ablation session, are connected by, for example, blue lines or rectangles 520. The lines 520 may be any shape, color or shade of color, or any shading. A line is one example, but any distinguishing feature such as a shape, shade, or color may be used to indicate a connection between electrodes (i.e., indicating area of ablation). The color of the lines (and/or shade of color or shading) 520 may be a different color (and/or shade or color or shading) than the color (and/or shade of color or shading) of the tags 510. The tag colors (and/or shade of color or shading) and/or the line colors (and/or shade of color or shading) of one ablation session may be different than the tag colors (and/or shade of color or shading) and/or lines colors (and/or shade of color or shading) of a different ablation session. The connecting of multiple tags together via lines 520 forms what may be referred to as a bracelet shape in this example of using a PFA non-focal lasso shaped catheter. However, if the ablation was performed by a different catheter, the connecting of the tags may form a different shape and may be referred differently. By connecting the tags, which represent positions of bipolar electrodes, showing that a PFA bipolar ablation application was performed between them, it helps to organize information, clear clutter on the screen, and visually separate ablation session from each other. This helps a physician identify which tags belong to a same ablation session. Using a catheter with bipolar electrodes (e.g. VARIPULSE^{™} catheter), ablation is performed at and between the electrodes, so the resulting bracelet visualizes areas where ablation energy was delivered in the proximity of ablation targets (e.g. energy was delivered or transferred between electrodes).

As shown in FIG. 5D, not all the tags 510 of an ablation session are connected by a line 520 (i.e. there is a gap 530 between two electrodes of a bracelet e.g., between distal and proximal electrodes in a ring-link catheter such as VARIPULSE^{™})). This may occur in a case of a bipolar PFA application in a PFA ablation session where a pair of electrodes were not part of the bipolar PFA application (e.g. there was no energy applied or transferred between that pair of electrodes). The gap 530 indicates an area where no energy was applied or transferred between that pair of electrodes.

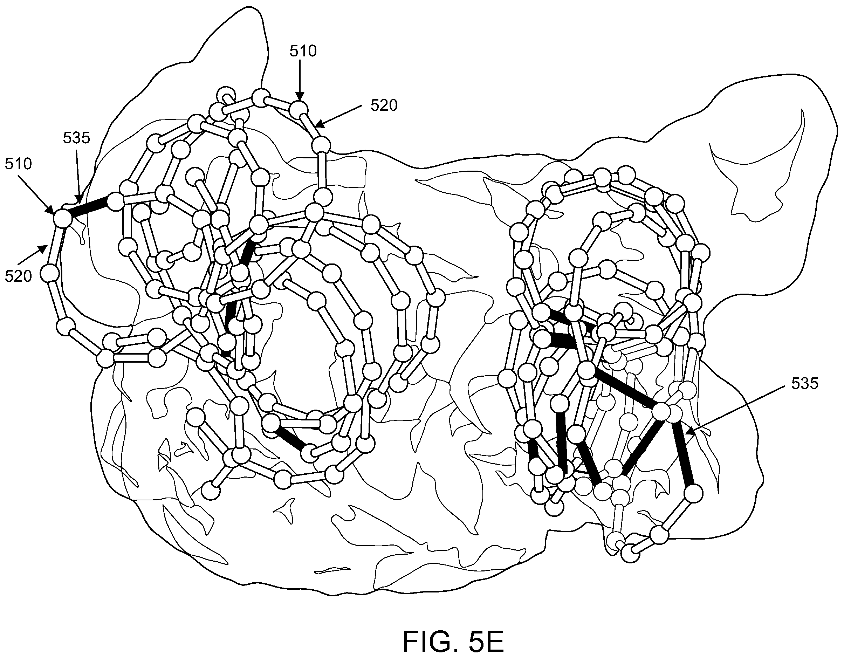

FIG. 5E is an example 3D visualization identifying areas between electrodes where no ablation application was performed. In FIG. 5E, a 3D point cloud of tags is shown that may be generated from a plurality of ablation sessions according to one or more embodiments. In FIG. 5E, a features (e.g. lines or filled in rectangles 535) are made/generated/produced/displayed in the gaps 530 between tags/electrodes 510 (e.g. circles) of an ablation session (i.e. bracelet), where these tags represent electrodes that were not part of a bipolar PFA application in a catheter's ablation electrodes (i.e. no ablation took place between those electrodes or no ablation energy was delivered in the proximity of ablation targets). In other words, the features (e.g., lines / filled in rectangles) 535 illustrate the gap in the bracelet. The features 535 that indicates a gap may be a different shape, color, shade of color and/or shading, than the lines or open (non-filled) rectangles 520 that indicate a connection of the tags 510 where an ablation occurred (i.e. bipolar PFA application was performed / energy was applied / ablation energy was delivered in the proximity of ablation targets). A line and rectangle are examples of a feature, but the feature indicating areas / gaps where no ablation took place may be any distinguishing feature such as a shape, color, shade, or shading. Additional information (not shown in FIG. 5E) may be displayed which may indicate, for example, a distance between electrodes (e.g. proximal and distal electrodes), an electrode's stability, or a number of PFA applications. The features 535 may indicate the most distal electrode at one end of the feature and the most proximal electrode at the other end of the feature of the catheter's ablation electrodes in the bracelet. The features 535 may indicate or identify any pair of electrodes that were not a part of a bipolar PFA application in a catheter's ablation electrodes and visualized in a bracelet visualization of an ablation session. The display of features 535 close the gap of the bracelet and provide a valuable clinical benefit of helping a physician to identify the area where an additional ablation is or may be needed to ensure the area isolation. This helps the physician identify how to manipulate (e.g. rotate) the catheter to ablate the areas that were not previously ablated.

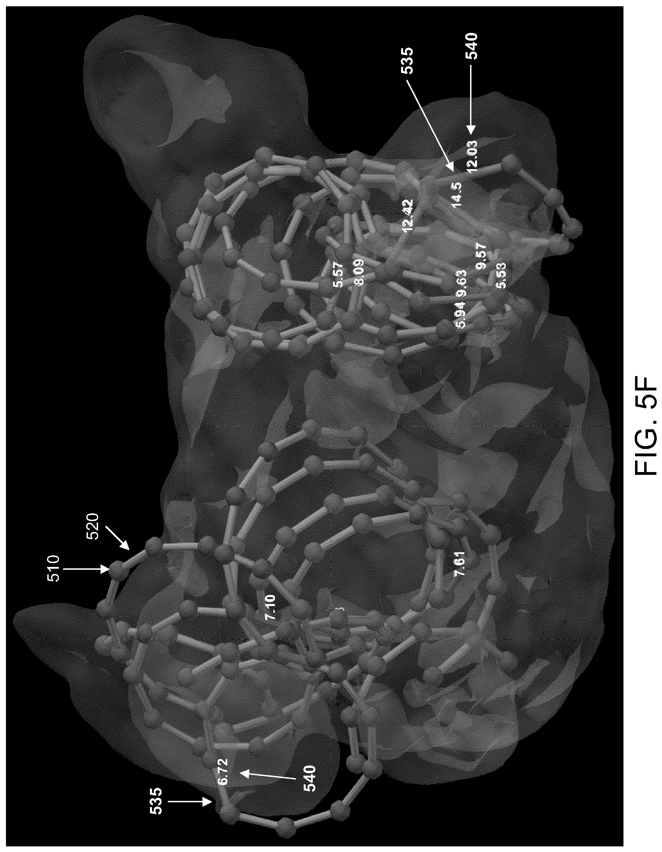

FIG. 5F is a screenshot of a 3D visualization identifying areas between electrodes where no ablation application was performed. In FIG. 5F, a 3D point cloud of tags is shown that may be generated from a plurality of ablation sessions according to one or more embodiments. In FIG. 5F, a feature (e.g. red lines or rectangles 535) are made/generated/produced/displayed in the gaps 530 between tags/electrodes (e.g., purple spheres or circles) 510 of an ablation session (i.e. bracelet), where these tags represent electrodes that were not part of a bipolar PFA application in a catheter's ablation electrodes (i.e. no ablation took place between those electrodes or ablation energy was delivered in the proximity of ablation targets). In other words, the features (e.g., red lines or rectangles) 535 illustrate the gap in the bracelet (i.e., indicate where no ablation took place). The features 535 that illustrate a gap may be a different shape, color, shade of color, or shading, than the features (e.g., blue lines or rectangles) 520 that indicate a connection of the tags/electrodes (e.g., purple sphere or circles) 510 where an ablation occurred (i.e. bipolar PFA application was performed / energy was applied / ablation energy was delivered in the proximity of ablation targets). A line and rectangle are examples of features, but the feature may be any distinguishing feature such as a shape, color, shade of color, or shading. Additional information 540 may be displayed (e.g. 6.72, 7.10, 7.61, 12.03, ...) which may indicate, for example, a distance between electrodes (e.g. proximal and distal electrodes), an electrode's stability, or a number of PFA applications. The features (e.g., red lines or rectangles) 535 may indicate a connection of the most distal electrode to the most proximal electrode of the catheter's ablation electrodes in the bracelet. The features (e.g., red lines or rectangles) 535 may indicate an area between any pair of electrodes that were not a part of a bipolar PFA application in a catheter's ablation electrodes and visualized in a bracelet visualization of an ablation session. The features (e.g., red lines or rectangles) 535 close the gap of the bracelet and provide a valuable clinical benefit of helping a physician to identify the area where an additional ablation is or may be needed to ensure the area isolation. This helps the physician identify how to manipulate (e.g. rotate) the catheter to ablate the areas that were not previously ablated.

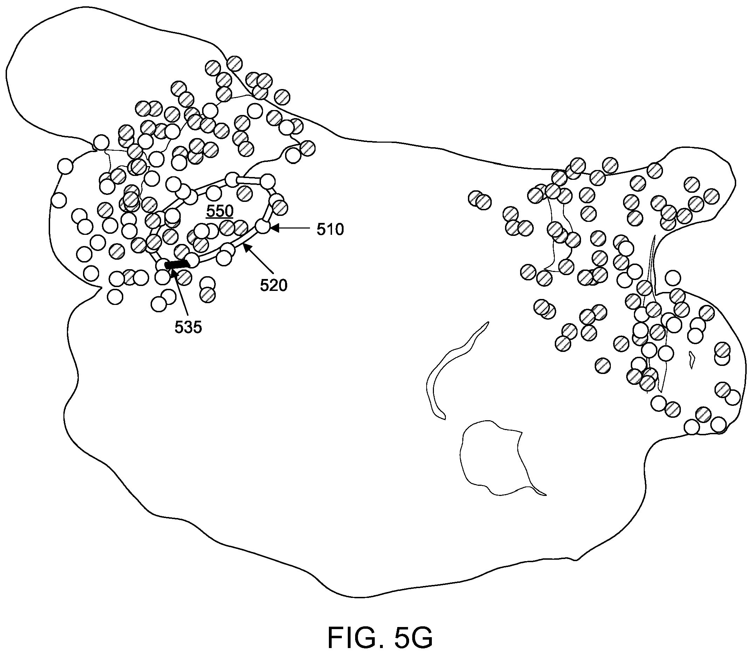

FIG. 5G shows an example 3D visualization identifying an ablation session (e.g., visualized as a bracelet shape). For example, a user (e.g. a physician or healthcare professional) may select one, or more than one, particular ring or bracelet (i.e. ablation session) to display. For example, in FIG. 5G, one ablation session (bracelet) 550 is displayed showing features 520 (e.g., lines or open rectangles) connecting features 510 (e.g., circles or spheres). Features 520 indicate areas between features 510 (i.e., electrodes) where ablation took place or where energy was delivered or ablation energy was delivered in the proximity of ablation targets. FIG. 5G also shows features 535 (e.g., lines or filed in rectangles) that indicates areas between electrodes where no ablation took place or where no energy was delivered or no ablation energy was delivered in the proximity of ablation targets. The features 510 in FIG. 5G (e.g., circles or spheres) comprise different colors or different levels of shading or cross-hatching. The different colors or different levels of shading or cross-hatching may indicate a level of PFA completion. The different colors or different levels of shading may indicate the number of applications completed, spatial stability or touch proximity index based on impedance (TPI), which may depend on, for example, the settings in the system (e.g. CARTO^{™} 3 System).

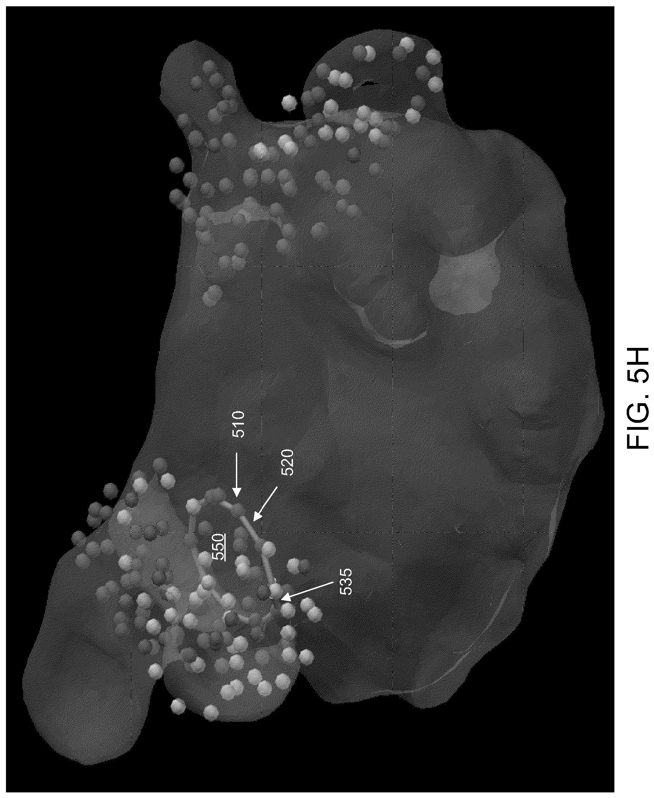

FIG. 5H shows a screenshot of a 3D visualization of an ablation session (e.g., bracelet). For example, a user (e.g. a physician or healthcare provider) may select one, or more than one, particular ring or bracelet (i.e. ablation session) to display. For example, in FIG. 5H, one ablation session (bracelet) 550 is displayed showing the features 520 (e.g., blue lines or rectangles) connecting features 510 (e.g., purple spheres or circles). Features 520 indicate areas between features 510 (i.e., electrodes) where ablation took place or where energy was delivered or ablation energy was delivered in the proximity of ablation targets. FIG.5H also shows features 535 (e.g., red line or rectangle) that indicates areas between electrodes where no ablation took place or where no energy was delivered or no ablation energy was delivered in the proximity of ablation targets. The features 510 in FIG. 5H (e.g., sphere or circles) comprise different colors or different levels of shading. The different colors or different levels of shading may indicate a level of PFA completion. The different colors or different levels of shading may indicate the number of applications completed, spatial stability or touch proximity index based on impedance (TPI), which may depend on, for example, the settings in the system (e.g. CARTO^{™} 3 System).

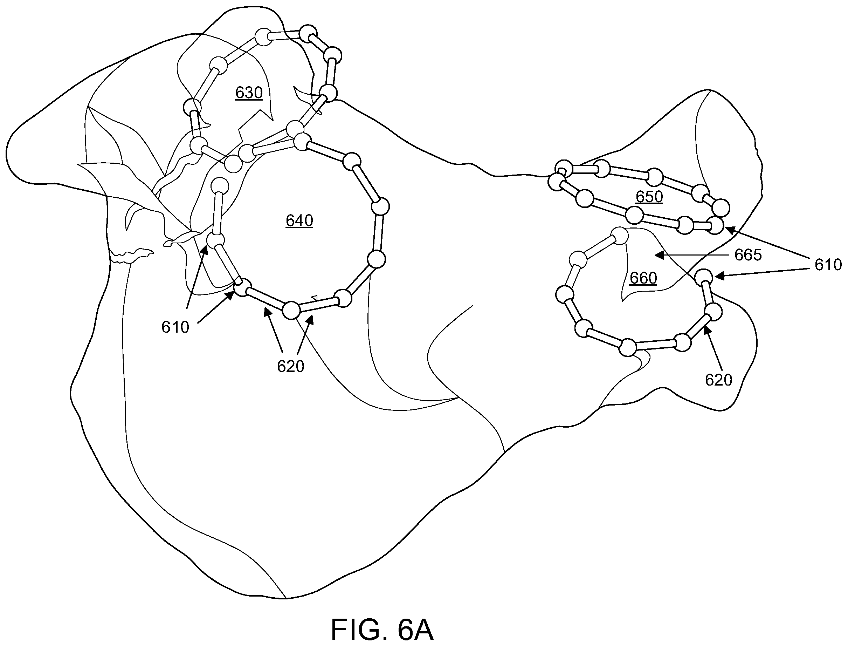

FIG. 6A is an example of a 3D visualization of a plurality of ablation sessions (e.g. visualized as bracelet shapes). In FIG. 6A, four bracelets are shown (630, 640, 650, and 660). Each bracelet represents a single ablation session. The ablation sessions may be performed using, for example, a variable lasso or looped shaped PFA multi-electrode non-focal ablation catheter, for example a VARIPULSE^{™} catheter. However, other non-focal catheters may be used. Each ablation session is represented by a plurality of features (tags / electrodes) 610 (e.g. spheres or circles) which are connected via features 620 (e.g. lines or open (non-filled) rectangles) forming, for example, a bracelet (e.g. 630, 640, 650, 660). The features 610 indicate positions of ablation electrodes that were part of an ablation application for each ablation session (e.g. a bipolar electrodes of a non-focal catheter).The bracelets show a gap between two features 610 (tags) (i.e. where there is no connecting feature 620), for example 665 in bracelet 660, which indicates that no ablation occurred (e.g. no energy was delivered or transferred) between those electrodes that are represented by the two tags. A bracelet indicates a single ablation session and indicates the position of electrodes (e.g. spheres or circles 610) and where ablation has taken place (e.g. area indicated by line/rectangle 620). This provides valuable clinical information helping a physician or healthcare provider to identify the areas that were ablated and where additional ablation is needed. The features 610 and the features 620 may be represented by distinguishing features such as, for example, a shape, color, shade of color, and/or shading.

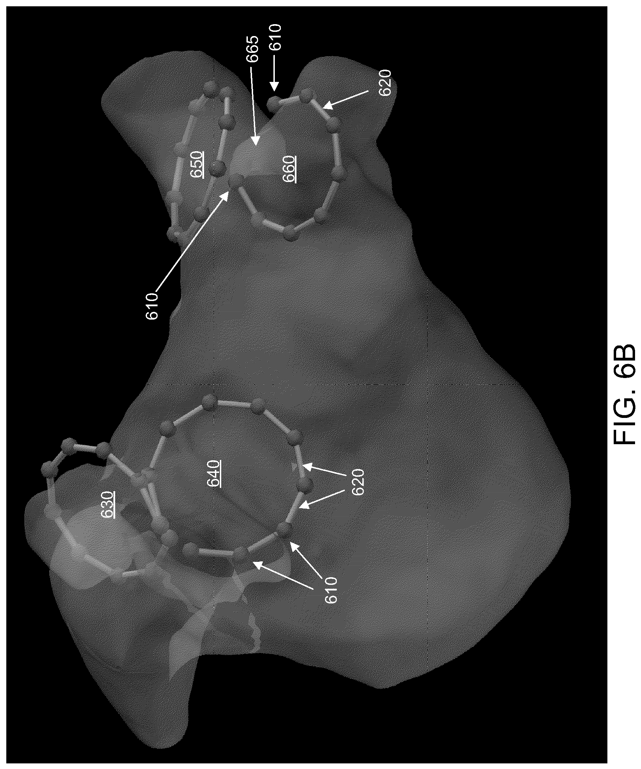

FIG. 6B is a screenshot of a 3D visualization of a plurality of ablation sessions (e.g. bracelets). In FIG. 6B, four bracelets are shown (630, 640, 650, and 660). Each bracelet represents a single ablation session. The ablation sessions may be performed using, for example, a variable lasso or looped shaped PFA multi-electrode non-focal ablation catheter, for example a VARIPULSE^{™} catheter. However, other non-focal catheters may be used. Each ablation session is represented by a plurality of features (tags /electrodes) 610 (e.g. purple spheres or circles) which are connected via features 620 (e.g. blue lines or rectangles) forming, for example, a bracelet (e.g. 630, 640, 650, 660). The features 610 indicate positions of ablation electrodes that were part of an ablation application for each ablation session (e.g. a bipolar electrodes of a non-focal catheter).The bracelets show a gap between two features 610 (tags) (i.e. where there is no connecting feature 620 (i.e., blue line)), for example 665 in bracelet 660, which indicates that no ablation occurred (e.g. no energy was delivered or transferred) between those electrodes that are represented by the two tags. A bracelet indicates a single ablation session and indicates the position of electrodes (e.g. purple spheres or circles) and where ablation has taken place (e.g. area indicated by the blue lines or rectangles 620). This provides valuable clinical information helping a physician or healthcare provider to identify the areas that were ablated and where additional ablation is needed. The features 610 and the features 620 may be represented by distinguishing features such as, for example, a shape, color, shade of color, and/or shading.

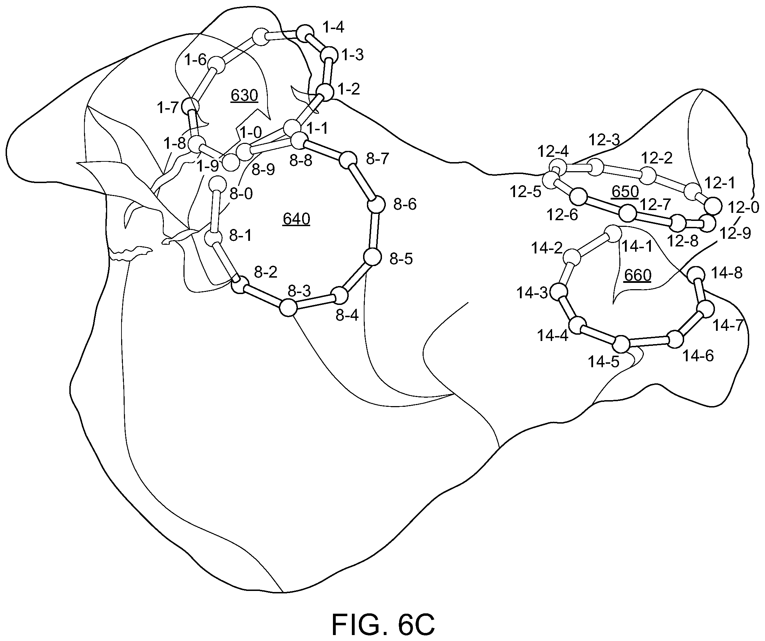

FIG. 6C is an example of FIG. 6A with additional information. Additional information may be displayed on or near the features 610 representing electrodes, features 620 representing areas of ablation, and/or features 635 representing areas of no ablation. The additional information may indicate for example, information regarding a count, an order, and/or a timing of an ablation session, and/or identification of a particular electrode. The additional information may be in any shape, color, size, and/or placement. The addition informational may indicate, for example, an ablation session and an electrode number. For example, bracelet 660 indicates 14-1, 14-2, 14-3, 14-4, 14-5, 14-6, 14-7, and 14-8. The 14-1 may indicate that the tag (circle or sphere) representing an electrode is the first electrode in the 14^{th} ablation session (bracelet). The 14-2 may indicate that the tag (circle sphere) representing an electrode is the second electrode in the 14^{th} ablation session (bracelet) and so on. Additional information may be any ablation related parameter (e.g., applications completed, spatial stability, TPI, count, order, timing, or identification of an electrode). The additional information may be color encoded on the indicator type number to facilitate a physician's comprehension.

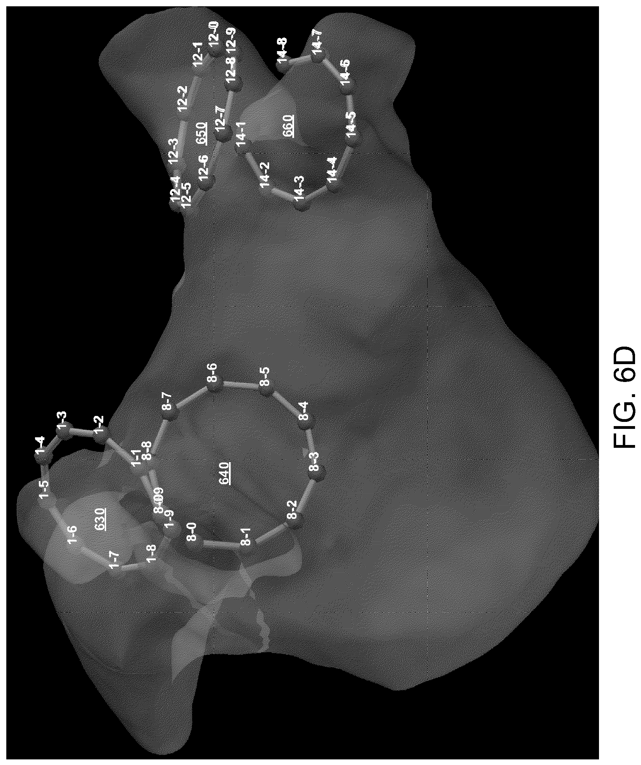

FIG. 6D is a screenshot of the example of FIG. 6B with additional information. Additional information may be on or near the features 610 representing electrodes, features 610 representing areas of ablation, and/or features 635 representing areas of no ablation. The additional information may indicate for example, information regarding a count, an order, and/or a timing of an ablation session, and/or identification of a particular electrode. The additional information is shown in white text in FIG. 6D, but may be in any color, size, and/or placement. The addition informational may indicate, for example, an ablation session and an electrode number. For example, bracelet 660 displays 14-1, 14-2, 14-3, 14-4, 14-5, 14-6, 14-7, and 14-8. The 14-1 may indicate that the tag (purple sphere or circle) representing an electrode is the first electrode in the 14^{th} ablation session (bracelet). The 14-2 may indicate that the tag (purple sphere or circle) representing an electrode is the second electrode in the 14^{th} ablation session (bracelet) and so on. Additional information may be any ablation related parameter (e.g., applications completed, spatial stability, TPI, count, order, timing, or identification of an electrode). The additional information may be color encoded on the indicator type number to facilitate a physician's comprehension.

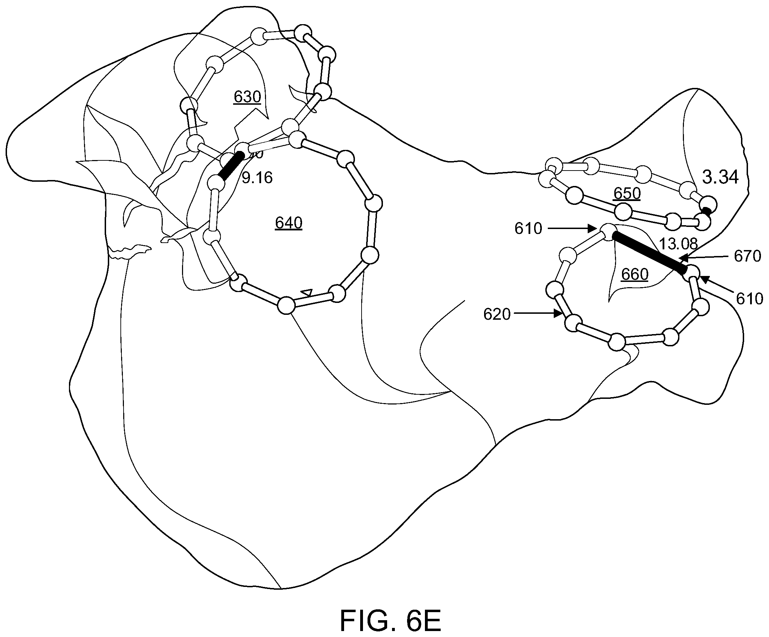

FIG. 6E is an example of FIG. 6A that includes a distinguishing indication of the gaps (i.e. where no ablation has taken place or no energy was delivered or transferred between electrodes) in the bracelets (e.g. during an ablation session that is represented by a bracelet for a ring-like VARIPULSE^{™} catheter). Feature 670 (dark line or filled in rectangle) in bracelets 640, 650, and 660 indicate an area (gap) between two tags 610 (i.e., circles) representing electrodes in a bracelet. However, any distinguishing feature to represent areas of no ablation may be used (e.g., a shape, color, shade of color, and/or shading) such that it is clearly different and/or distinguishable (e.g. different shape, color, shade of color, and/or shading) than the features 620 (i.e. non-filled lines or rectangles) that indicate ablation and is clearly different and/or distinguishable (e.g. different shape, color, shade of color, and/or shading) than the features 610 (i.e. spheres or circles) representing electrodes. The features 670 (i.e., filled in lines or rectangles) provide valuable clinical information helping a physician or healthcare provider to identify the areas where no ablation occurred (i.e., no energy was delivered) and where additional ablation is needed. Additional information may be provided (e.g., "9.16", "3.34", "13.08) as shown in FIG. 6E next to the features 670. For example, the additional information may be a distance between two tags (electrodes). For example, for bracelet 660, the "13.08" may indicate a distance (e.g. in mm) between the two electrodes 610 that are connected by feature 670. The additional information may be in any shape, color, size, and/or placement.

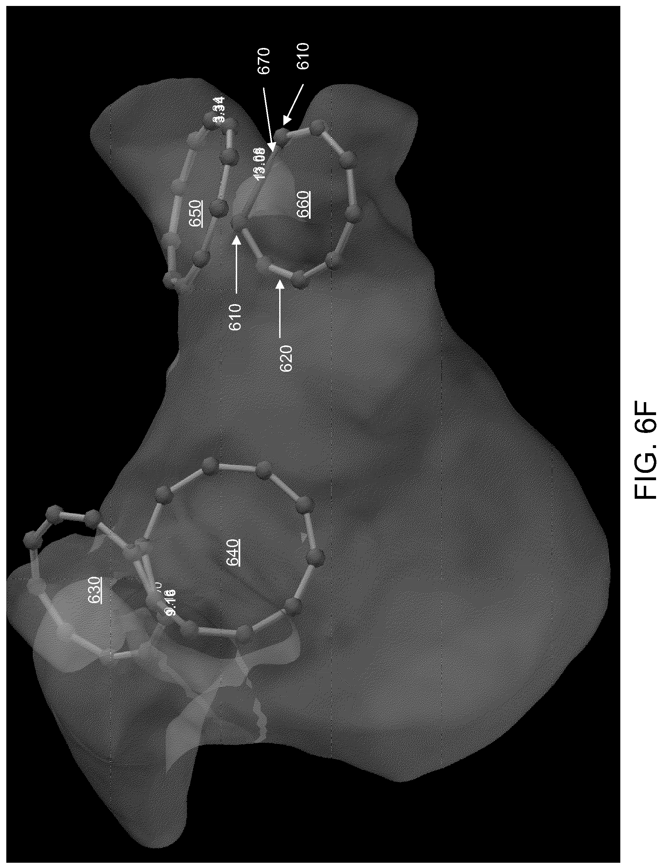

FIG. 6F is a screenshot of the example of FIG. 6B that includes a distinguishing indication of the gaps (i.e. where no ablation has taken place or no energy was delivered or transferred between electrodes) in the bracelets (e.g. during an ablation session that is represented by a bracelet for a ring-like VARIPULSE^{™} catheter). Feature 670 (red line or rectangle) in bracelets 640, 650, and 660 indicate an area (gap) between two tags 610 (i.e. purple spheres or circles) representing electrodes in a bracelet. However, any distinguishing feature to represent areas of no ablation may be used (e.g., a shape, color, shade of color, and/or shading) such that it is clearly different and/or distinguishable (e.g. different shape, color, shade of color, and/or shading) than the features 620 (i.e. blue lines) that indicate ablation and is clearly different and/or distinguishable than features 610 (i.e. purple spheres or circles) representing electrodes. The features 670 (i.e., red lines or rectangles) provide valuable clinical information helping a physician or healthcare provider to identify the areas where no ablation occurred (i.e., no energy was delivered) and where additional ablation is needed. Additional information may be provided (e.g., "9.16", "3.34", "13.08) as shown in FIG. 6F next to the red lines/rectangles. For example, the additional information may be a distance between two tags (electrodes). For example, for bracelet 660, the "13.08" may indicate a distance (e.g. in mm) between the two electrodes 610 that are connected by the red line 670. The additional information is shown in white text in FIG. 6F, but may be in any shape, color, size, and/or placement.

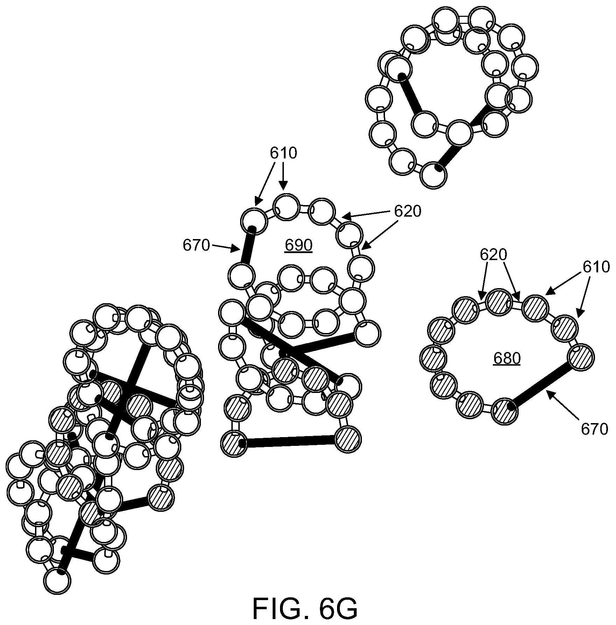

FIG. 6G is an example 3D visualization of ablation sessions that includes an indication identifying a gap between tags / electrodes (i.e. where no ablation has taken place or no energy was delivered or transferred between electrodes) in the bracelets (e.g. representing an ablation session). Each ablation session is represented by a plurality of tags 610 (e.g. spheres or circles) connected via features 620 (e.g. lines or non-filled rectangles) forming, for example, a bracelet (e.g. 680, 690). A feature 670 (e.g., black lines or filled in rectangles) indicate areas (gaps) between two tags 610 (i.e. no energy was delivered between electrodes) in a bracelet. This feature 670 indicates that no energy was delivered (i.e., no ablation occurred) between the electrodes (tags). However, any distinguishing feature to represent areas of no ablation 670 may be used (e.g., a shape, color, shade of color, and/or shading) such that it is clearly different and/or distinguishable (e.g. different color, shape, shade of color, and/or shading) than the features 620 (i.e. non-filled lines or rectangles) that indicate ablation and is clearly different and/or distinguishable (e.g. different shape, color, shade of color, and/or shading) than the features 610 (i.e. spheres or circles) representing electrodes. The features 670 provide valuable clinical information helping a physician or healthcare professional to identify the areas where no ablation occurred and where additional ablation is or may be needed. The features 610 in FIG. 6G (e.g., circles or spheres) comprise different colors or different levels of shading or cross-hatching. The different colors or different levels of shading or cross-hatching may indicate a level of PFA completion. The different colors or different levels of shading may indicate the number of applications completed, spatial stability or touch proximity index based on impedance (TPI), which may depend on, for example, the settings in the system (e.g. CARTO^{™} 3 System).

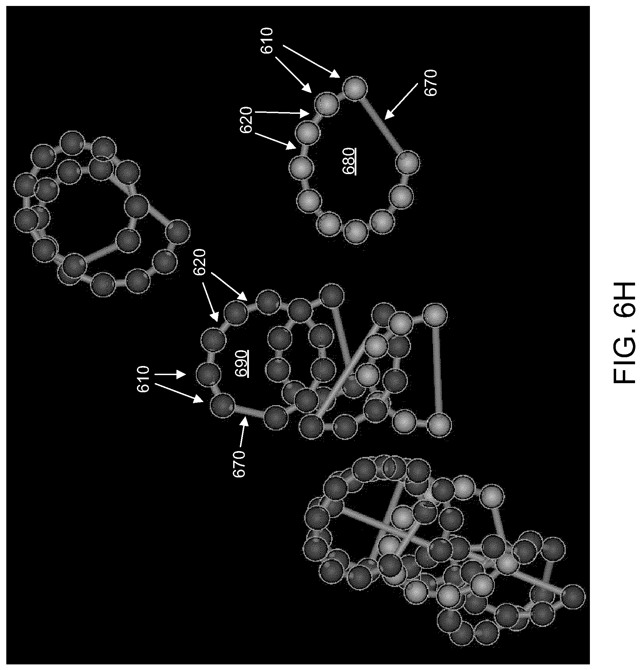

FIG. 6H is a screenshot of a 3D visualization of ablation sessions that includes an indication identifying a gap between tags / electrodes (i.e. where no ablation has taken place or no energy was delivered or transferred between electrodes) in the bracelets (e.g. representing an ablation session). Each ablation session is represented by a plurality of tags 610 (e.g. spheres or circles) connected via features 620 (e.g. blue lines or rectangles) forming, for example, a bracelet (e.g. 680, 690). A feature 670 (e.g., orange lines or rectangles) indicate areas (gaps) between two tags 610 (i.e. no energy was delivered between electrodes) in a bracelet. This feature 670 indicates that no energy was delivered (i.e., no ablation occurred) between the electrodes (tags). However, any distinguishing feature to represent areas of no ablation 670 may be used (e.g., a shape, color, shade of color, and/or shading) such that it is clearly different and/or distinguishable (e.g. different color, shape, shade of color, and/or shading) than the features 620 (i.e. blue lines or rectangles) that indicate ablation and is clearly different and/or distinguishable (e.g. different shape, color, shade of color, and/or shading) than the features 610 (i.e. spheres or circles) representing electrodes. The features 670 provide valuable clinical information helping a physician or healthcare professional to identify the areas where no ablation occurred and where additional ablation is or may be needed. The features 610 in FIG. 6H (e.g., circles or spheres) comprise different colors or different levels of shading or cross-hatching. The different colors or different levels of shading or cross-hatching may indicate a level of PFA completion. The different colors or different levels of shading may indicate the number of applications completed, spatial stability or touch proximity index based on impedance (TPI), which may depend on, for example, the settings in the system (e.g. CARTO^{™} 3 System)..

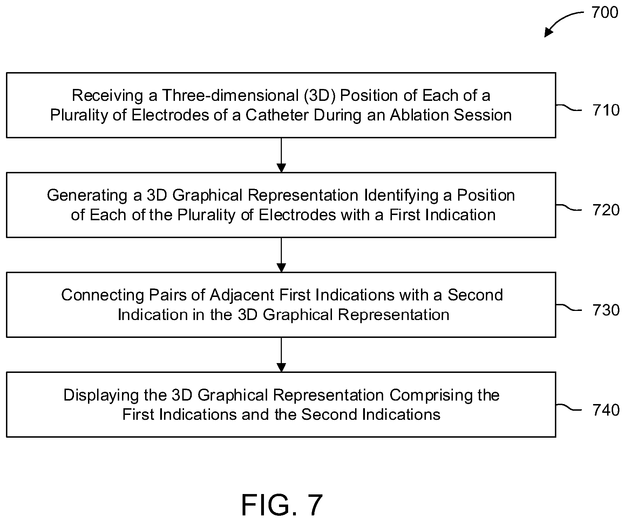

FIG. 7 shows an example method 700 for generating display of information of ablation sessions. An ablation session may be bipolar ablation where energy is delivered between electrodes. A processor may be configured to receive, from one or more sensors, a three-dimensional (3D) position of each of a plurality of electrodes of a therapeutic catheter during an ablation session 710. The catheter may be a bipolar Pulsed-Field Ablation (PFA) catheter. The catheter may be a non-focal catheter. The catheter may have a complex topology. For example, the catheter may have a lasso or loop shape. For example, the catheter may have a flower or basket shape. The catheter may have bipolar electrodes. The catheter may be a VARIPULSE^{™} catheter. The processor may be configured to receive a three-dimensional (3D) position of each of a plurality of electrodes of a catheter for a plurality of ablation sessions.

The processor may be configured to generate a 3D graphical representation that identifies a position of each of the plurality of electrodes with a first indication 720. The first indication may be a tag (e.g., CARTO VISITAG^{™} Module Tag). The first indication may be represented by at least a first distinguishing feature such as a first graphic shape (e.g. a sphere, a dot, circle, square, rectangle), a first shade or shading, and/or a first color. For example, the first indication may be a purple sphere or circle (510, 610) as shown in FIGs. 5B, 5D, 5F, 5H, 6B, 6D, 6F, and 6H.

The processor may be configured to connect pairs of adjacent first indications with a second indication in the 3D graphical representation 730. The second indications may indicate locations between electrodes where energy was delivered (i.e. an ablation took place or ablation energy was delivered in the proximity of ablation targets). The second indications may be represented by at least a second distinguishing feature such as, for example, a second graphic (e.g. a solid line, dotted line, dashed line, rectangle, or any other shape), a second shade or shading, and/or a second color. A second distinguishing feature (e.g. second color) used for the second indications may be different than a first distinguishing feature (e.g. the first color) that is used for the first indications. For example, the second indications may be a solid blue line (520, 620) as shown in FIGs. 5D, 5F,5H, 6B, 6D, 6F or a solid orange line as shown in FIG. 6H. The 3D graphical representation may be in the shape of a ring or bracelet and may be referred to as a bracelet. The 3D graphical representation may be in a different shape depending on the type of catheter used. The bracelet may comprise a gap, with no second indication between a pair of first indications (i.e. tags / electrodes) where no energy was delivered between them (i.e., no ablation took place or no ablation energy was delivered in the proximity of ablation targets). If no energy was delivered between a pair of electrodes, then an ablation did not take place.

The processor may be configured to display, on a display device or monitor, the bracelet comprising the first indications and the second indications 740.

The steps 710-730 may be performed for each of a plurality of ablation sessions of an ablation procedure. The processor may be configured to display a 3D graphical representation (i.e. bracelet) for each ablation session, so that the display shows a plurality of bracelets. The display of the bracelets helps a physician identify which tags (i.e. electrodes) belong to a same ablation session, which helps the physician identify positions of electrodes and areas that were ablated and areas that need to be ablated. For example, the processor may be configured to display the bracelets for all the ablations sessions. For example, if there are ten ablation sessions, then the processor may be configured to display ten bracelets corresponding to the ten ablation sessions. The processor may be configured to selectively display one or more particular bracelets. For example, a user (e.g. a physician) may select which bracelet or bracelets that they want displayed and the processor may be configured to display the selected bracelet or bracelets. For example, the processor may determine which bracelet or bracelets to display. Bracelets may be uniquely identified by alphanumeric, color, shading, visual or other identifiers.

The processor may be configured to generate additional information. Additional information may include any ablation related parameter (e.g., applications completed, spatial stability, TPI, count, order, timing, identification of an electrode,...). The processor may be configured to add the additional information to one or more bracelets. The processor may be configured to display the additional information on a display device or monitor. The additional information may be on or near the first indications and/or second indications. The additional information may be displayed, for example, as shown in white text in FIG. 6B. The additional information may be of any color, size, and/or placement. The addition information may indicate, for example, an ablation session and an electrode number. For example, bracelet 660 of FIG. 6D indicates 14-1, 14-2, 14-3, 14-4, 14-5, 14-6, 14-7, and 14-8. The 14-1 may indicate that the tag (purple sphere) representing an electrode is the first electrode in the 14^{th} ablation session (bracelet). The 14-2 may indicate that the tag (purple sphere) representing an electrode is the second electrode in the 14^{th} ablation session (bracelet) and so on. The physician may use the additional information to, for example, turn on and off, individually, each set of connector lines in the 3D graphical representation. This may allow the physician to review specific ablation sessions within an overall procedure and in what order and at what time they performed during the procedure

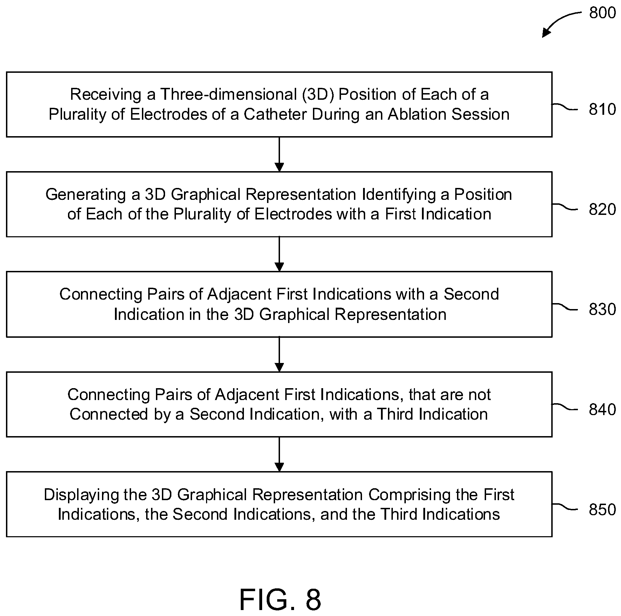

FIG. 8 shows an example method 800 for generating display of information of ablation sessions. An ablation session may be bipolar ablation where energy is delivered between electrodes. A processor may be configured to receive, from one or more sensors, a three-dimensional (3D) position of each of a plurality of electrodes of a therapeutic catheter during an ablation session 810. The catheter may be a bipolar Pulsed-Field Ablation (PFA) catheter. The catheter may be a non-focal catheter. The catheter may have a complex topology. For example, the catheter may have a lasso or loop shape. For example, the catheter may have a flower or basket shape. The catheter may have bipolar electrodes. The catheter may be a VARIPULSE^{™} catheter. The processor may be configured to receive a three-dimensional (3D) position of each of a plurality of electrodes of a catheter for a plurality of ablation sessions.

The processor may be configured to generate a 3D graphical representation that identifies a position of each of the plurality of electrodes with a first indication 820. The first indication may be a tag (e.g.,CARTO VISITAG^{™} Module Tag). The first indication may be represented by at least a first distinguishing feature such as a first graphic shape (e.g. a sphere, a dot, circle, square, rectangle, or any shape), a first shade, shading and/or a first color. For example, the first indication may be a purple sphere or circle (510, 610) as shown in FIGs. 5B, 5D, 5F, 5F, 6B, 6D, 6F, and 6H.

The processor may be configured to connect pairs of adjacent first indications with a second indication in the 3D graphical representation 830. The second indications may indicate locations between electrodes where energy was delivered (i.e. an ablation took place or ablation energy was delivered in the proximity of ablation targets). The second indications may be represented by at least a second distinguishing feature such as, for example, a second graphic shape (e.g. a solid line, dotted line, dashed line, rectangle, or any other graphic), a second shade, shading, and/or a second color. A second distinguishing feature (e.g. a second color) used for the second indications may be different than a first distinguishing feature (e.g. the first color) that is used for the first indications. For example, the second indications may be a solid blue line (520, 620) as shown in FIGs. 5D, 5F, 5G, 6B, 6D, 6F or a solid orange line as shown in FIG. 6H. The 3D graphical representation may be in the shape of a ring or bracelet and may be referred to as a bracelet. The 3D graphical representation may be a different shape depending on the type of catheter used. The bracelet may comprise a gap, with no second indication between a pair of first indications (i.e. tags / electrodes) where no energy was delivered between them (i.e., no ablation took place or no ablation energy was delivered in the proximity of ablation targets). If no energy was delivered between a pair of electrodes, then an ablation did not take place.

The processor may be configured to identify or visualize the pair of adjacent first indications, which are not connected with a second indication (i.e. a gap), with a third indication 840. The third indication may indicate a location where no ablation took place (i.e. no energy was delivered between the electrodes represented by the pair of first indications). The third indication may be represented by at least a third distinguishing feature such as, for example, a third graphic shape (e.g. a solid line, dotted line, dashed line, rectangle, or any other graphic), a third shade, shading, and/or a third color. A third distinguishing feature (e.g. a third color) may be different than the first color that is used for the first indications and different than the second color that is used for the second indications. The third indication may be the same size or thickness as the second indications. The third indication may be different size or thickness as the second indications. For example, the third indications may be a solid red line (535) as shown in FIGs. 5F, (670) as shown in FIG. 6F, or solid orange line (670) as shown in FIG. 6H.

The processor may be configured to display, on a display device or monitor, the bracelet comprising the first indications, the second indications, and the third indications 850.

The steps 810-840 may be performed for each of a plurality of ablation sessions of an ablation procedure. The processor may be configured to display a 3D graphical representation (i.e. bracelet) for each ablation session, so that the display shows a plurality of bracelets. The display of the bracelets helps a physician identify which tags (i.e. electrodes) belong to the same ablation session, which helps the physician identify positions of electrodes and areas that were ablated and areas that need to be ablated. For example, the processor may be configured to display the bracelets for all the ablations sessions. For example, if there are ten ablation sessions, then the processor may be configured to display ten bracelets corresponding to the ten ablation sessions. The processor may be configured to selectively display one or more particular bracelets. For example, a user (e.g. a physician) may select which bracelet or bracelets that they want displayed and the processor may be configured to display the selected bracelet or bracelets. For example, the processor may determine which bracelet or bracelets to display.

The processor may be configured to generate additional information. Additional information may include, for example, information regarding a count, an order, a timing of an ablation session, and/or distance between tags (i.e. electrodes). The processor may be configured to add the additional information to one or more bracelets. The processor may be configured to display additional information on a display device or monitor. The additional information may be on or near the first indications, second indications, and/or third indications. The additional information may be of any color, size, and/or placement.

Additional information may be displayed, for example, shown in white text in FIG. 6B and FIG. 6C. The addition information may indicate, for example, an ablation session and an electrode number. For example, bracelet 660 of FIG. 6DB indicates 14-1, 14-2, 14-3, 14-4, 14-5, 14-6, 14-7, and 14-8. The 14-1 may indicate that the tag (purple sphere) representing an electrode is the first electrode in the 14^{th} ablation session (bracelet). The 14-2 may indicate that the tag (purple sphere) representing an electrode is the second electrode in the 14^{th} ablation session (bracelet) and so on. Additional information may indicate a distance between a pair of tags (i.e. electrodes). The physician may use the additional information to, for example, turn on and off, individually, each set of connector lines in the 3D graphical representation. This may allow the physician to review specific ablation sessions within an overall procedure and in what order and at what time they performed during the procedure.

Although features and elements are described above in particular combinations, one of ordinary skills in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A method for generating display of information of an ablation session, the method comprising:
receiving, by a processor from one or more sensors, a three-dimensional (3D) position information of each of a plurality of electrodes (420, 430, 440) of a catheter (410) during the ablation session;
generating, by the processor, a 3D graphical representation identifying a position of each of the plurality of electrodes with a first indication (510, 610);
connecting, by the processor, pairs of adjacent first indications with a second indication (520, 620) in the 3D graphical representation, wherein the second indications indicate locations between electrodes where ablation energy was delivered; and
displaying, by the processor on a display device, the 3D graphical representation comprising the first indications and the second indications.

2. The method of claim 1, further comprising:
connecting pairs of adjacent first indications, that are not connected by a second indication, with a third indication (535, 670), wherein the third indications indicate locations between electrodes where ablation energy was not delivered; and
displaying, by the processor on the display device, the 3D graphical representation comprising the first indications, the second indications, and the third indications.

3. The method of claim 2, wherein the first indications are represented by at least a first distinguishing feature, the second indications are represented by at least a second distinguishing feature, and the third indications are represented by at least a third distinguishing feature, wherein the first distinguishing feature is a first color, wherein the second distinguishing feature is a second color, and wherein the third distinguishing feature is a third color, and wherein the first color, the second color, and the third color are different.

4. The method of either one of claims 1-3, wherein the 3D graphical representation comprises a gap between a pair of electrodes where no ablation energy was delivered, wherein the pair of electrodes does not have a second indication connecting the pair of electrodes.

5. The method of either one of claims 1-4, wherein the catheter is a non-focal multi-electrode catheter, wherein the catheter is a Pulsed-Field Ablation (PFA) catheter, and wherein the first indication is a sphere and the second indication is a line.

6. The method of either one of claims 1-5, wherein the electrodes are bipolar electrodes.

7. The method of either one of claims 1-6, further comprising:
generating additional information; and
displaying the additional information along with the 3D graphical representation;
wherein the additional information comprises at least one of: a count of an ablation session, an order of an ablation session, a timing of an ablation session, or a distance between a pair of electrodes.

8. A system for generating display of information of an ablation session, the system comprising:
a device comprising a processor in communication with one or more sensors and a catheter (410) comprising a plurality of electrodes (420, 430, 440); and
wherein the processor is configured to receive, from the one or more sensors, three-dimensional (3D) position information of each of a plurality of electrodes of a catheter during the ablation session;
wherein the processor is further configured to generate a 3D graphical representation identifying a position of each of the plurality of electrodes with a first indication (510, 610);
wherein the processor is further configured to connect pairs of adjacent first indications with a second indication (520, 620) in the 3D graphical representation, wherein the second indications indicate locations between electrodes where ablation energy was delivered; and
wherein the processor is further configured to display, by the processor on a display device, the 3D graphical representation comprising the first indications and the second indications.

9. The system of claim 8, wherein:
the processor is further configured to connect pairs of adjacent first indications, that are not connected by a second indication, with a third indication (535, 670), wherein the third indications indicate locations between electrodes where ablation energy was not delivered; and
the processor is further configured to display, by the processor on the display device, the 3D graphical representation comprising the first indications, the second indications, and the third indications.

10. The system of claim 9, wherein the first indications are represented by at least a first distinguishing feature, the second indications are represented by at least a second distinguishing feature, and the third indications are represented by at least a third distinguishing feature, wherein the first distinguishing feature is a first color, wherein the second distinguishing feature is a second color, and wherein the third distinguishing feature is a third color, and wherein the first color, the second color, and the third color are different.

11. The system of either one of claims 8-10, wherein the 3D graphical representation comprises a gap between a pair of electrodes where no ablation energy was delivered, wherein the pair of electrodes does not have a second indication connecting the pair of electrodes.

12. The system of either one of claims 8-11, wherein the catheter is a non-focal multi-electrode catheter, wherein the catheter is a Pulsed-Field Ablation (PFA) catheter, and wherein the first indication is a sphere and the second indication is a line.

13. The system of either one of claims 8-12, wherein the electrodes are bipolar electrodes.

14. The system of either one of claims 8-13, wherein:
the processor is further configured to generate additional information; and
the processor is further configured to display the additional information along with the 3D graphical representation;
wherein the additional information comprises at least one of: a count of an ablation session, an order of an ablation session, a timing of an ablation session, or a distance between a pair of electrodes..

15. The system of either one of claims 8-14, wherein:
the processor is further configured to receive, from a user, information that indicates which 3D graphical representation, from a plurality of generated 3D graphical representations, to display; and
the processor is further configured to selectively display the indicated 3D graphical representation.
